# EUROPÄISCHE PATENTSCHRIFT

(11) **EP 0 684 795 B1**
(45) Veröffentlichungstag und Bekanntmachung des Hinweises auf die Patenterteilung: **05.01.2000**
(21) Anmeldenummer: 94906872.0
(22) Anmeldetag: 15.02.1994
(51) Int. Cl.: A61B 19/00, A61F 2/30

(54) **VORRICHTUNG UND VERFAHREN ZUR VORBEREITUNG UND UNTERSTÜTZUNG CHIRURGISCHER EINGRIFFE**
DEVICE AND PROCESS FOR PREPARING AND SUPPORTING SURGICAL OPERATIONS
DISPOSITIF ET PROCEDE POUR LA PREPARATION ET LE SOUTIEN D'INTERVENTIONS CHIRURGICALES

(30) Priorität: 16.02.1993 DE 4304570
(43) Veröffentlichungstag der Anmeldung: 06.12.1995
(73) Patentinhaber: Kliegis, Ulrich Georg, D-24106 Kiel (DE)
(72) Erfinder: KLIEGIS, Ulrich, D-24106 Kiel (DE)
(74) Vertreter: Heldt, Gert, Dr. Dipl.-Ing.
(86) Internationale Anmeldenummer: DE9400156
(87) Internationale Veröffentlichungsnummer: WO9418899

(56) Entgegenhaltungen:
- EP-A- 0 097 001
- EP-A- 0 456 103
- EP-A- 0 469 966
- WO-A-91/04711

## Beschreibung

Die Erfindung betrifft eine Vorrichtung und ein Verfahren zur Vorbereitung und Unterstützung chirurgischer Eingriffe, insbesondere zur Vorbereitung und Unterstützung von Knochentransplantationen.

Ein Beispiel derartigen Vorrichtungen bzw. Verfahren ist in WO-A-91/04711 beschrieben.

Bei einer Transplantation eines Knochenstücks am Schädel als Beispiel für eine Knochentransplantation wird nach der Entfernung des krankhaft veränderten oder inoperabel zerstörten Knochenstücks vorzugsweise im Bereich des Beckenkamms ein Transplantat entnommen, welches anschließend durch Abtragen von Material an seinen Rändern bearbeitet wird, uni seine äußere Gestalt der Form der Schädelöffnung möglichst weitgehend anzupassen, so daß es ohne größere, den Heilungsprozeß verzögernde Randfugen in die Schädelöffnung eingesetzt werden kann.

Die Bearbeitung des Transplantats erfolgt dabei bisher von Hand und erfordert vom Chirurgen großes handwerkliches Geschick und ein gutes räumliches Vorstellungsvermögen, um in wenigen Bearbeitungsschritten ein gut passendes Transplantat herzustellen. Bei weniger erfahrenen oder geübten Chirurgen ist diese Vorgehensweise jedoch zum einen sehr zeitaufwendig, da sie ein häufiges vergleichen des Transplantats mit der Schädelöffnung erfordert, um zu verhindern, daß zuviel Material abgetragen wird. Zum anderen wird von diesen meist eine verhältnismäßig einfache geometrische Form als Umriß für die Schädelöffnung bzw. für das Transplantat gewählt, um das Anpassen des Transplantats zu vereinfachen, was jedoch zur Folge hat, daß an Schädel häufig mehr gesundes Knochengewebe entfernt wird, als erforderlich, und daß demzufolge auch ein größeres Transplantat entnommen werden muß, wodurch der Heilungsprozeß an der Entnahmestelle des Transplantat verlangsamt wird.

Ausgehend hiervon liegt der Erfindung die Aufgabe zugrunde, ein Verfahren und eine Vorrichtung der eingangs angegebenen Art zu entwickeln, die es ermöglichen, ein Transplantat unabhängig von dessen Form mit hoher Genauigkeit einer Transplantationsstelle anzupassen.

Diese Aufgabe wird im Hinblick auf die Vorrichtung erfindungsgemäß durch den Gegenstand des Anspruchs 1 gelöst und, im Hinblick auf das Verfahren, durch den Gegenstand des Anspruchs 14.

Die Erfindung sieht dabei vor, daß mit der Positioniereinrichtung mindestens eine Halterung verbindbar ist, auf welcher das Transplantat lösbar befestigt ist. Die Positioniereinrichtung kann ohne Entfernung der Halterung sowohl eingesetzt werden, um das Transplantat bei dessen Bearbeitung mit hoher Genauigkeit rechnergestützt gegenüber einem feststehenden Bearbeitungswerkzeug, beispielsweise einem Fräskopf zu bewegen, als auch zum Positionieren des fertig bearbeiteten Transplantats an der Transplantationsstelle, bis es dort befestigt ist. Alternativ dazu kann die Halterung mit dem lösbar befestigten Transplantat so ausgebildet sein, daß sie sich sowohl wahlweise mit der Positioniereinrichtung als auch stationär in einer vorgegebenen oder ausmeßbaren geometrischen Beziehung zum Bezugssystem befestigen läßt, so daß sie zuerst in stationärem Zustand mit Hilfe eines mit der Positioniereinrichtung verbundenen Bearbeitungswerkzeugs in die vorgesehene Form gebracht und anschließend nach Austausch des Bearbeitungswerkzeugs gegen die Halterung mit Hilfe der Positioniereinrichtung positioniert werden kann.

Um sowohl bei der Positionierung als auch bei der Formgebung eine hohe Positionier- bzw. Bearbeitungsgenauigkeit zu gewährleisten, ist gemäß einer weiteren bevorzugten Ausgestaltung der Erfindung eine Datenverarbeitungsanlage vorgesehen, die einen Speicher aufweist, in welchem ein Datensatz gespeichert ist, der in einem dreidimensionalen Koordinatensystem die individuelle anatomische Morphologie der Transplantationsstelle wiedergibt, die gemäß einer weiteren bevorzugten Ausgestaltung der Erfindung präoperativ durch Auswertung der Ergebnisse von nichtinvasiven Untersuchungen, beispielsweise von Computertomographie-, Kernspintomographie-, Ultraschall-, Röntgen- oder hobgraphischen Untersuchungen ermittelt wurde. Bei diesen Untersuchungen wird vorteilhaft die zu untersuchende Körperregion durch zueinander parallele Schnittaufnahmen mit ausreichender Auflösung schichtweise erfasst und die gewonnenen Daten in der Datenverarbeitungsanlage ausgewertet, um daraus eine dreidimensionale Strukturabbildung der Knochen oder Organe im Bereich des vorgesehenen Operationsfelds herzustellen, die als Grundlage für die Planung und Simulation des Eingriffs dienen kann. Die Kenntnis der Morphologie des Gewebes in Operationsfeld erleichtert zum einen die Planung der Osteotomielinien und ist zum anderen nach Festlegen derselben auch bei der Suche nach einer passenden Entnahmestelle für das Transplantat hilfreich, da die Suche mit Hilfe der Datenverarbeitungsanlage die Anzahl der überprüfbaren Optionen immens erhöht.

Gemäß einer weiteren bevorzugten Ausgestaltung der Erfindung wird die Positioniereinrichtung von der Datenverarbeitungsanlage auf der Grundlage des dort gespeicherten dreidimensionalen Datensatzes gesteuert, dessen Koordinatensystem mit dem Bezugssystem, in dem die Bewegungen der Positioniereinrichtung erfolgen, im Hinblick auf den Koordinatennullpunkt, die Anzahl und Ausrichtung der Koordinatenachsen zueinander und den Maßstab zur Deckung gebracht wird. Dies kann mit Hilfe einer mechanischen, akustischen oder optischen Meßvorrichtung erfolgen, mit der vor oder während dem Eingriff die Koordinaten charakteristischer festliegender Punkte des Operationsfelds gegenüber einem Bezugspunkt vermessen werden, der in einer bekannten geometrischen Beziehung zum Koordinatennullpunkt des Manipulatorarms steht. Dasselbe gilt für die Vermessung der freiliegenden Oberflächen des verbleibenden gesunden Knochens oder Gewebes nach Entfernen der krankhaft veränderten oder verletzten Teile.

Intraoperativ kann mit dieser Meßvorrichtung auch die räumliche Gestalt des auf der stationär befestigten Halterung angeordneten Transplantats vor dessen Bearbeitung bezüglich des Bezugssystems vermessen und ein mit der Positioniereinrichtung verbundenes Bearbeitungswerkzeug nach Korrelation der Koordinatensysteme durch die Datenverarbeitungsanlage entlang der zuvor ausgemessenen Oberflächen des verbleibenden gesunden Knochens geführt werden, die dem Transplantat dabei synthetisch überlagert werden.

Die Positioniereinrichtung weist gemäß einer vorteilhaften Ausgestaltung der Erfindung mehrere Freiheitsgrade der Bewegung auf, so daß das mit ihr verbundene chirurgische Instrument, das Bearbeitungswerkzeug oder die Halterung für das Transplantat jede beliebige Raumlage einnehmen können.

Gemäß einer weiteren vorteilhaften Ausgestaltung der Erfindung weist die Positioniereinrichtung mindestens einen Manipulatorarm auf, der im Bereich seines freien Endes eine Aufnahme aufweist, in die das chirurgische Instrument, das Bearbeitungswerkzeug oder die Transplantathalterung eingesetzt werden können.

Dabei können vorteilhafterweise mehrere Manipulatorarme vorgesehen sein, mit deren Aufnahmen Sensoren zum Bestimmen biologischer oder physikalischer Parameter, wie beispielsweise von Muskel- oder Hirnströmen oder des pH-Werts im Bereich des Operationsfeldes, oder chirurgische Hilfsinstrumente, z.B. Haken oder dergleichen verbindbar sind, welche das Operationsfeld ermüdungsfrei offenhalten und durch Ansteuern der Manipulatorarme mit hoher Präzision verschoben werden können.

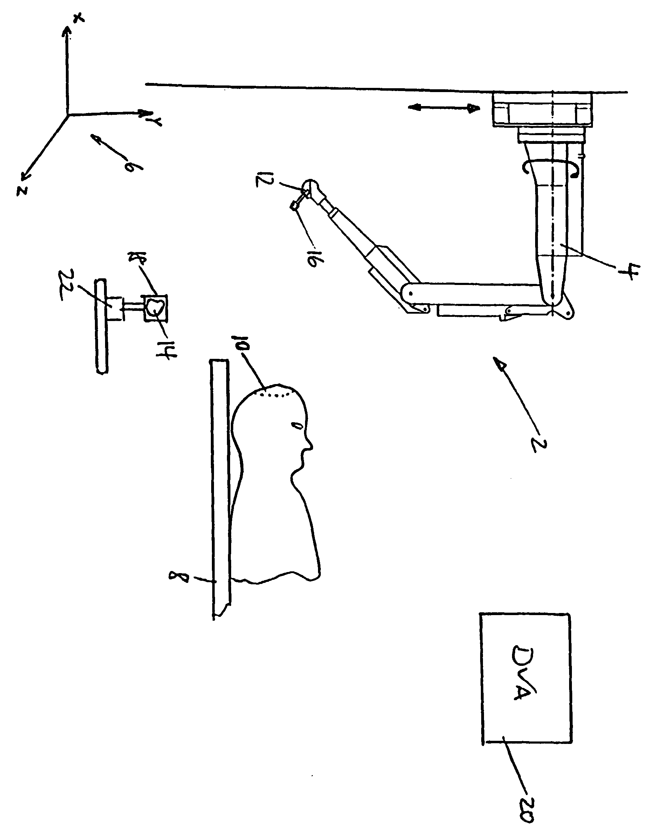

Im folgenden wird die Erfindung anhand eines in der Zeichnung in schematischer Weise dargestellten Ausführungsbeispiels näher erläutert. Dabei zeigt die einzige Figur eine Positioniereinrichtung einer erfindungsgemäßen Vorrichtung zur Unterstützung chirurgischer Eingriffe.

Die in der Zeichnung dargestellte Positioniereinrichtung 2 besteht im wesentlichen aus einem numerisch gesteuerten Manipulatorarm 4, der mit mehreren Freiheitsgraden der Bewegung gegenüber einem stationären Bezugssystem 6 bewegt werden kann. Auf einer ebenfalls stationären Operationsauflage 8 kann ein Patient so fixiert werden, daß sich ein vorgesehenes Operationsfeld 10 im Bewegungsbereich einer am freien Ende des Manipulatorarms 4 angeordneten Aufnahme 12 befindet, in welche wahlweise ein chirurgisches Instrument, ein Bearbeitungswerkzeug 16 oder eine Halterung 18 für ein Transplantat 14 einsetzbar ist.

Die Bewegungen des Manipulatorarms 4 und damit die Bewegungen des in die Aufnahme 12 eingesetzten Instruments, des Bearbeitungswerkzeugs 16 oder der Halterung 18 werden von einer Datenverarbeitungsanlage (DVA) 20 unmittelbar oder mittelbar durch Übertragung von Daten auf eine numerische Steuerung des Manipulatorarms 4 selbst gesteuert.

Die DVA 20 besitzt einen Speicher, in welchem ein 3D-Datensatz für eine dreidimensionale Strukturabbildung des Operationsfeldes 10 gespeichert ist, welche in einem eigenen Koordinatensystem die individuelle anatomische Morphologie der Transplantationsstelle enthält, die präoperativ durch Auswertung der Ergebnisse von nichtinvasiven Untersuchungen, beispielsweise von Computertomographie-, Kernspintomographie-, Ultraschall-, Röntgen- oder holographischen Untersuchungen ermittelt wurde, und Grundlage für die Planung der Osteotomielinien war, die ebenfalls im Speicher der DVA 20 gespeichert sind.

Eine nicht dargestellte optische oder mechanische Vermessungseinrichtung dient dazu, nach dem Fixieren des Patienten auf der Operationsauflage 8 die Koordinaten charakteristischer Punkte des Operationsfeldes 10, beispielsweise leicht erkennbare Knochenmerkmale in Bezug zum stationären Bezugssystem 6 zu erfassen. Die in die DVA 20 eingelesenen Meßdaten werden dort mit dem Datensatz der Strukturabbildung verglichen und das Koordinatensystem der Strukturabbildung mit dem Bezugssystem 6 rechnerisch zur Deckung gebracht, so dag die Strukturabbildung und die geplanten Osteotomielinien dem Operationsfeld 10 überlagert werden können. Dadurch können beispielsweise nach Beendigung der Osteotomie durch den Chirurgen mit Hilfe eines in die Aufnahme 12 eingesetzten Bearbeitungswerkzeugs 16, beispielsweise eines rotierend angetriebenen Fräskopfs, die das Transplantat 14 umgebenden Knochenoberflächen einer Feinbearbeitung unterzogen werden, um sie mit den gespeicherten Osteotomielinien exakt zur Deckung zu bringen.

Die Vermessungseinrichtung dient weiter zum Vermessen des auf der Halterung 18 lösbar befestigten Transplantats 14. Die Halterung 18, die eine Form ähnlich einer kleinen Osteosyntheseplatte aufweist, wird dazu in einer gegenüber der Operationsauflage 8 stationären Befestigungsvorrichtung 22 befestigt, die ebenfalls im Bewegungsbereich eines in die Aufnahme 12 des Manipulatorarms 4 eingesetzten Bearbeitungswerkzeugs 16 angeordnet ist. Anschließend werden die Koordinaten der Begrenzungsflächen des Transplantats 14 bestimmt und dem Transplantat 14 rechnerisch die geometrische Gestalt der zuvor vermessenen Oberflächen des verbleibenden gesunden Gewebes oder Knochens überlagert, entlang derer dann von der DVA 20 die Schneid- oder Fräskante des in die Aufnahme 12 eingesetzten Bearbeitungswerkzeugs 16 geführt wird, um die Begrenzungsflächen des Transplantats 14 möglichst genau den Oberflächen des das Transplantat 14 umgebenden Gewebes oder Knochens anzupassen. Die Bewegung des Manipulatorarms 4 kann dabei allerdings so gesteuert werden, daß später zwischen Transplantat 14 und dem umgebenden Gewebe oder Knochen stellenweise eine kleine gleichmäßig starke Fuge verbleibt, die mit Knochenzement gefüllt werden kann, um das Transplantat 14 unverschiebbar zu fixieren.

Nach der Formgebung des Transplantats 14 wird dieses dann zusammen mit der Halterung 18 in die Aufnahme 12 eingesetzt und im Operationsfeld 10 an der vorgesehenen Transplantationsstelle in korrekter Ausrichtung positioniert, wobei die Bewegung des Manipulatorarms 4 durch die DVA 20 gesteuert wird.

Die Positioniereinrichtung 2 kann weitere numerisch gesteuerte Manipulatorarme aufweisen, in deren Aufnahmen während des Eingriffs Sensoren oder chirurgische Hilfsinstrumente wie Haken eingesetzt werden, um Informationen über den jeweiligen Status biologisch relevanter Parameter, beispielsweise die Muskelströme oder Hirnströme vom Operationsfeld zu einem physiologischen Überwachungssystem zu übermitteln bzw. um das Operationsfeld ermüdungsfrei offenzuhalten.

## Patentansprüche

1. Vorrichtung zur Vorbereitung und Unterstützung chirurgischer Eingriffe mit einer in einer vorgegebenen oder vermeßbaren geometrischen Beziehung zu einem Operationsfeld (10) angeordneten numerisch gesteuerten Positioniereinrichtung (2) die gegenüber dem Operationsfeld (10) bewegbar ist, dadurch gekennzeichnet, daß mit der Positioniereinrichtung (2) mindestens eine Halterung (18) für ein mit ihr lösbar verbindbares Transplantat (14) lösbar verbindbar und gegenüber dem Operationsfeld (10) bewegbar ist, das mit der Halterung (18) lösbar verbundene Transplantat (14) hinsichtlich seiner Begrenzungsflächen mit einem Bearbeitungswerkzeug (16) entsprechend einer geometrischen Gestalt bearbeitbar ist, die von zuvor vermessenen Oberflächen eines eine Transplantationsstelle umgebenden verbleibenden Gewebes vorgegeben ist, und das Transplantat (14) im Operationsfeld (10) an der vorgesehenen Transplantationsstelle positionierbar ist.

2. Vorrichtung nach Anspruch 1, gekennzeichnet durch eine Vermessungseinrichtung zum Vermessen von Koordinaten des Operationsfeldes (10) gegenüber einem stationären Bezugssystem.

3. Vorrichtung nach einem der Ansprüche 1 oder 2, dadurch gekennzeichnet, daß die Halterung (18) wahlweise mit der Positioniereinrichtung (2) verbindbar oder an einer bezüglich des Bezugssystems (6) stationären Befestigungsvorrichtung (22) lösbar befestigbar ist.

4. Vorrichtung nach Anspruch 3, dadurch gekennzeichnet, daß die Befestigungsvorrichtung (22) in einer vorgegebenen oder vermeßbaren geometrischen Beziehung zum Operationsfeld (10) angeordnet ist.

5. Vorrichtung nach einem der Ansprüche 1 bis 4, gekennzeichnet durch eine die Positioniereinrichtung (2) steuernde Datenverarbeitungsanlage (20) mit einem Speicher.

6. Vorrichtung nach Anspruch 5, dadurch gekennzeichnet, daß im Speicher der Datenverarbeitungsanlage (20) ein Datensatz für eine dreidimensionale Strukturabbildung des Operationsfeldes (10) gespeichert ist.

7. Vorrichtung nach Anspruch 5 oder 6, dadurch gekennzeichnet, daß im Speicher der Datenverarbeitungsanlage (20) ein Datensatz für eine dreidimensionale Abbildung von geplanten Osteotomielinien gespeichert ist.

8. Vorrichtung nach einem der Ansprüche 1 bis 7, dadurch gekennzeichnet, daß die Positioniereinrichtung (2) mehrere Freiheitgrade der Bewegung aufweist.

9. Vorrichtung nach einem der Ansprüche 1 bis 8, dadurch gekennzeichnet, daß die Positioniereinrichtung (2) mindestens einen Manipulatorarm (4) aufweist.

10. Vorrichtung nach Anspruch 9, gekennzeichnet durch eine im Bereich eines freien Endes des Manipulatorarms (4) angeordnete Aufnahme (12).

11. Vorrichtung nach einem der Ansprüche 1 bis 10, dadurch gekennzeichnet, daß das mit der Positioniereinrichtung (2) verbundene Bearbeitungswerkzeug (16) motorisch antreibbar ist.

12. Vorrichtung nach Anspruch 11, dadurch gekennzeichnet, daß das Bearbeitungswerkzeug (16) ein spanabhebendes Werkzeug, ein Fräswerkzeug oder ein Bohrwerkzeug ist.

13. Vorrichtung nach einem der Ansprüche 1 bis 11, dadurch gekennzeichnet, daß die Halterung (18) für das Transplantat in Form einer Osteosyntheseplatte ausgebildet ist.

14. Verfahren zur Vorbereitung und Unterstützung chirurgischer Eingriffe, mit dem präoperativ die individuelle anatomische Morphologie im Bereich eines vorgebenen Operationsfeldes (10) ermittelt wird und dabei ermittelte Morphologiedaten als Grundlage für eine Planung und/oder Simulationen des Eingriffs sowie für eine numerische Steuerung mindestens einer gegenüber dem Operationsfeld (10) beweglichen Positioniereinrichtung (2) aufbereitet werden, dadurch gekennzeichnet, daß mit der Positioniereinrichtung (2) eine Halterung (18) für ein mit ihr lösbar verbundenes Transplantat (14) lösbar verbunden wird, so daß das mit der Halterung (18) lösbar verbundene Transplantat (14) entlang seiner Begrenzungsflächen mit einem Bearbeitungswerkzeug (16) entsprechend einer geometrischen Gestalt bearbeitbar und positionierbar ist, die von zuvor vermessenen Oberflächen eines eine Transplantationsstelle umgebenden verbleibenden Gewebes vorgegeben ist.

15. Verfahren nach Anspruch 14, dadurch gekennzeichnet, daß die Morphologiedaten durch nichtinvasive Untersuchungsverfahren ermittelt werden.

16. Verfahren nach Anspruch 14 oder 15, dadurch gekennzeichnet, daß die Morphologiedaten durch Computertomographie-, Kernspinresonanz-, Röntgen-, Ultraschall- und/oder holographische Untersuchungen ermittelt werden.

17. Verfahren nach Anspruch 15 oder 16, dadurch gekennzeichnet, daß die Morphologiedaten aus Serien hintereinander angeordneter Schnittaufnahmen des Operationsfelds (10) ermittelt werden.

18. Verfahren nach einem der Ansprüche 14 bis 17, dadurch gekennzeichnet, daß aus den ermittelten Morphologiedaten eine dreidimensionale Abbildung des Operationsfeldes (10) erstellt wird.

19. Verfahren nach Anspruch 18, dadurch gekennzeichnet, daß die Abbildung der Planung von Osteotomielinien zugrundegelegt wird.

20. Verfahren nach Anspruch 18 oder 19, dadurch gekennzeichnet, daß die Koordinaten der Osteotomielinien in Steuerbefehle für die Positioniereinrichtung (2) übernommen werden.

## Claims

1. Apparatus for preparing for and assisting in surgical interventions comprising a numerically controlled positioning device (2) which is arranged in a predetermined or measurable geometrical relationship with an operative area (10) and which is movable with respect to the operative area (10), characterised in that at least one holder (18) for a transplant (14) which can be releasably connected to the holder can be releasably connected to the positioning device (2) and is movable with respect to the operative area (10), the transplant (14) which can be releasably connected to the holder (18) can be treated in regard to its boundary surfaces with a treatment tool (16) in accordance with a geometrical configuration which is predetermined by previously measured surfaces of a remaining tissue surrounding a transplant location, and the transplant (14) can be positioned in the operative area (10) at the intended transplant location.

2. Apparatus according to claim 1 characterised by a measuring device for measuring co-ordinates of the operative area (10) with respect to a stationary reference system.

3. Apparatus according to one of claims 1 and 2 characterised in that the holder (18) can be selectively connected to the positioning device (2) or releasably fixed to a fixing device (22) which is stationary with respect to the reference system (6).

4. Apparatus according to claim 3 characterised in that the fixing device (22) is arranged in a predetermined or measurable geometrical relationship with the operative area (10).

5. Apparatus according to one of claims 1 to 4 characterised by a data processing installation (20) having a memory, for controlling the positioning device (2).

6. Apparatus according to claim 5 characterised in that stored in the memory of the data processing installation (20) is a data set for a three-dimensional structural representation of the operative area (10).

7. Apparatus according to claim 5 or claim 6 characterised in that stored in the memory of the data processing installation (20) is a data set for a three-dimensional representation of planned osteotomy lines.

8. Apparatus according to one of claims 1 to 7 characterised in that the positioning device (2) has a plurality of degrees of freedom of movement.

9. Apparatus according to one of claims 1 to 8 characterised in that the positioning device (2) has at least one manipulator arm (4).

10. Apparatus according to claim 9 characterised by a mounting (12) arranged in the region of a free end of the manipulator arm (4).

11. Apparatus according to one of claims 1 to 10 characterised in that the treatment tool (16) connected to the positioning device (2) is drivable by motor means.

12. Apparatus according to claim 11 characterised in that the treatment tool (16) is a cutting tool, a milling tool or a drilling tool.

13. Apparatus according to one of claims 1 to 11 characterised in that the holder (18) for the transplant is in the form of an osteosynthesis plate.

14. A method of preparing for and assisting in surgical interventions, with which pre-operatively the individual anatomic morphology in the region of an intended operative area (10) is ascertained and morphology data ascertained in that procedure are prepared as a basis for planning and/or simulation of the intervention and for numerical control of at least one positioning device (2) which is movable with respect to the operative area (10), characterised in that a holder (18) for a transplant (14) which is releasably connected to the holder is releasably connected to the positioning device (2) so that the transplant (14) which is releasably connected to the holder (18) can be treated with a treatment tool (16) and positioned along its boundary surfaces in accordance with a geometrical configuration which is predetermined by previously measured surfaces of a remaining tissue surrounding a transplant location.

15. A method according to claim 14 characterised in that the morphology data are ascertained by non-invasive investigation procedures.

16. A method according to claim 14 or claim 15 characterised in that the morphology data are ascertained by computer tomography, nuclear magnetic resonance, X-ray, ultrasonic and/or holographic investigations.

17. A method according to claim 15 or claim 16 characterised in that the morphology data are ascertained from series of successively arranged slice scans of the operative area (10).

18. A method according to one of claims 14 to 17 characterised in that a three-dimensional representation of the operative area (10) is produced from the ascertained morphology data.

19. A method according to claim 18 characterised in that the representation forms the basis for planning of osteotomy lines.

20. A method according to claim 18 or claim 19 characterised in that the coordinates of the osteotomy lines are transferred into control commands for the positioning device (2).

## Revendications

1. Dispositif de préparation et d'assistance pour des interventions chirurgicales, comprenant un système de positionnement à commande numérique (2) disposé dans une relation géométrique prédéterminée ou mesurable par rapport à un champ opératoire (10), qui peut être déplacé par rapport audit champ opératoire (10), caractérisé par le fait qu'au moins un support (18) pour un transplant (14) lié de manière séparable audit support peut être fixé de manière démontable au système de positionnement (2) et déplacé par rapport au champ opératoire (10), que le transplant (14) lié de manière séparable au support (18) peut être usiné au niveau de ses surfaces extérieures conformément à une forme géométrique déterminée par des surfaces préalablement mesurées d'un tissu entourant un emplacement de transplantation et que le transplant (14) peut être positionné à l'emplacement de transplantation prévu dans le champ opératoire (10).

2. Dispositif selon la revendication 1, caractérisé par un dispositif de mesure pour mesurer les coordonnées du champ opératoire (10) par rapport à un système de référence stationnaire.

3. Dispositif selon une des revendications 1 ou 2, caractérisé par le fait que le support (18) peut être lié de manière sélective au système de positionnement (2) ou peut être fixé de manière séparable à un dispositif de fixation (22) stationnaire par rapport au système de référence (6).

4. Dispositif selon la revendication 3, caractérisé par le fait que le dispositif de fixation (22) est disposé dans une relation géométrique prédéterminée ou mesurable par rapport au champ opératoire (10).

5. Dispositif selon une des revendications 1 à 4, caractérisé par une installation de traitement de données (20) avec une mémoire, qui commande le système de positionnement (2).

6. Dispositif selon la revendication 5, caractérisé par le fait qu'un jeu de données pour une représentation structurelle en trois dimensions du champ opératoire (10) est mémorisé dans la mémoire de l'installation de traitement de données (20).

7. Dispositif selon la revendication 5 ou 6, caractérisé par le fait qu'un jeu de données pour une représentation en trois dimensions de lignes d'ostéotomie prévues est mémorisé dans la mémoire de l'installation de traitement de données (20).

8. Dispositif selon une des revendications 1 à 7, caractérisé par le fait que le système de positionnement (2) présente plusieurs degrés de liberté de déplacement.

9. Dispositif selon une des revendications 1 à 8, caractérisé par le fait que le système de positionnement (2) comporte au moins un bras de manipulateur (4).

10. Dispositif selon la revendication 9, caractérisé par un moyen de réception (12) disposé dans la région d'une extrémité libre du bras de manipulateur (4).

11. Dispositif selon une des revendications 1 à 10, caractérisé par le fait que l'outil de traitement (16) lié au système de positionnement (2) peut être entrainé par moteur.

12. Dispositif selon la revendication 11, caractérisé par le fait que l'outil de traitement (16) est un outil à enlèvement de copeaux, une fraise ou un foret.

13. Dispositif selon une des revendications 1 à 11, caractérisé par le fait que le support (18) pour le transplant est réalisé sous la forme d'une plaque d'ostéosynthèse.

14. Procédé de préparation et d'assistance pour des interventions chirurgicales, à l'aide duquel on détermine dans une phase préopératoire la morphologie anatomique individuelle dans la région d'un champ opératoire (10) préétabli et on traite les données de morphologie déterminées comme élément de base pour une planification et/ou des simulations de l'intervention ainsi que pour une commande numérique d'au moins un système de positionnement (2) mobile par rapport au champ opératoire (10), caractérisé par le fait qu'au moins un support (18) pour un transplant (14) lié de manière séparable audit support est fixé de manière démontable au système de positionnement (2), de telle sorte que le transplant (14) lié de manière séparable au support (18) puisse être usiné à l'aide d'un outil d'usinage (16) au niveau de ses surfaces extérieures et positionné en accord avec une forme géométrique qui est déterminée par des surfaces préalablement mesurées d'un tissu entourant un emplacement de transplantation.

15. Procédé selon la revendication 14, caractérisé par le fait que l'on détermine les données de morphologie parune méthode d'examen non invasive.

16. Procédé selon la revendication 14 ou 15, caractérisé par le fait que l'on détermine les données de morphologie par des examens par scanographie, par résonance magnétique nucléaire, par radiographie, par échographie ou par holographie.

17. Procédé selon la revendication 15 ou 16, caractérisé par le fait que l'on détermine les données de morphologie à partir de séries de clichés successifs de plans du champ opératoire (10).

18. Procédé selon une des revendications 14 à 17, caractérisé par le fait que l'on établit une représentation en trois dimensions du champ opératoire à partir des données de morphologie déterminées.

19. Procédé selon la revendication 18, caractérisé par le fait que l'on utilise la représentation pour la planification des lignes d'ostéotomie.

20. Procédé selon une des revendications 18 ou 19, caractérisé par le fait que l'on intègre les coordonnées des lignes d'ostéotomie dans des ordres de commande pour le système de positionnement (2).
